(19) Europäisches Patentamt / European Patent Office / Office européen des brevets

(11) **EP 3 192 022 B1**

(12) **EUROPEAN PATENT SPECIFICATION**

(45) Date of publication and mention
of the grant of the patent:
**17.04.2019 Bulletin 2019/16**

(21) Application number: **15766608.2**

(22) Date of filing: **30.07.2015**

(51) Int Cl.:
**G06Q 10/06** *(2012.01)*        **G06Q 50/22** *(2018.01)*

(86) International application number:
**PCT/IB2015/055755**

(87) International publication number:
**WO 2016/020803 (11.02.2016 Gazette 2016/06)**

(54) **A SYSTEM FOR CHECKING A CORRECT ORAL HYGIENE PROCEDURE**

SYSTEM ZUR ÜBERPRÜFUNG EINER KORREKTEN MUNDHYGIENEPROZEDUR

SYSTÈME DE VÉRIFICATION D'UNE PROCÉDURE D'HYGIÈNE BUCCALE CORRECTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.08.2014 IT BG20140033**

(43) Date of publication of application:
**19.07.2017 Bulletin 2017/29**

(73) Proprietor: **Sarubbo, Davide
87020 Tortora Marina (CS) (IT)**

(72) Inventors:
• **SARUBBO, Davide
87020 Tortora Marina (CS) (IT)**
• **MARCON, Marco
I-20021 Bollate (MI) (IT)**

(74) Representative: **Perrotta, Aldo
Ing. Aldo Perrotta
Corso Umberto 81
88068 Soverato (IT)**

(56) References cited:
**US-A1- 2010 091 112**

• **Anna Flagg ET AL: "AN INTELLIGENT
TOOTHBRUSH: Machines for Smart Brushing", ,
5 June 2011 (2011-06-05), XP055184189, Toronto,
Canada Retrieved from the Internet:
URL:http://www.annaflagg.com/AnIntelligent
Toothbrush(Flagg).pdf [retrieved on 2015-04-20]**
• **YU-CHEN CHANG ET AL: "Playful toothbrush",
PROCEEDING OF THE TWENTY-SIXTH ANNUAL
CHI CONFERENCE ON HUMAN FACTORS IN
COMPUTING SYSTEMS , CHI '08, 5 April 2008
(2008-04-05), page 363, XP055184192, DOI:
10.1145/1357054.1357115 ISBN:
978-1-60-558011-1**

EP 3 192 022 B1

**Description**

The present invention relates to a system for checking a correct oral hygiene procedure, to the related method and to the related computer program.

Educating children in correct oral hygiene is currently a difficult task for the educator, in view of the low attractiveness of daily dental cleaning for children. Even using special toothbrushes depicting their favourite cartoon characters or using toothpastes with inviting colours and flavours do not succeed, in most cases, in inducing the right amount of care and attention in children in performing a repetitive activity, nor yet in guaranteeing that said activity is performed with the right methods and times. Said activity is often performed incorrectly even by adults who, due to being in a hurry or for other contingent reasons, may perform the procedure hastily and incorrectly.

Prior art systems for checking a correct oral hygiene procedure are disclosed in Anna Flagg ET AL: "An Intelligent Toothbrush: Machines for Smart Brushing", Rehabilitation Engineering and Assistive Technology Society of North America (RESNA), Toronto, Canada, June 5-8, 2011 and in Yu-Chen Chang ET AL: "Playful Toothbrush: UbiComp Technology for Teaching Tooth Brushing to Kindergarten Children", 26th Annual CHI Conference on Human Factors in Computing Systems, CHI 2008 : conference proceedings, April 5-10, 2008, Florence, Italy.

The object of the present invention is providing a system for checking a correct oral hygiene procedure.

Another object is providing a system which is interactive.

A further object is providing a system which is also appealing and amusing at the same time.

According to the present invention, these objects and others are achieved by a system for checking a correct oral hygiene procedure according to claim 1.

Said objects are also achieved by a method for checking a correct oral hygiene procedure according to claim 6.

Said objects are also achieved by a computer program adapted to perform the method of claim 6.

Further characteristics of the invention are described in the dependent claims.

This solution has various advantages with respect to the solutions of the prior art.

The proposed system is therefore intended to provided an auxiliary tool for the educator to ensure children clean their teeth correctly in an interactive way, possibly with a cartoon character, enjoying themselves and simultaneously learning the importance of this daily task. The system is therefore also an auxiliary tool for an adult in checking that the brushing technique and times dedicated to oral hygiene are correct.

The system is therefore aimed, through use of manual toothbrushes provided with opportune markers which may be recognised and located by the portable device, at analysing the motion of the toothbrush with respect to the position of the face, assessing correct use and the time dedicated to oral hygiene.

The widespread use of portable computers of reduced dimensions, such as tablets, notebooks or smartphones, allows their easy positioning in the bathroom for the period necessary to brush the teeth. In particular, the proposed system makes use of said devices to allow complete interaction between the user, the toothbrush and the application provided for analysis of correct use and education in correct oral hygiene, despite other devices with similar data acquisition and processing capacity also being available.

Said system also allows, through appropriate following of a target, the position of the toothbrush in space to be located and the motion with respect to the user's mouth to be analysed.

It is therefore possible, using simple video cameras provided on smartphones and tablets, to perform precise analysis of a correct oral hygiene procedure and correct any errors committed by the user.

In order to make the application more attractive to younger user, it is also possible, due to the fact that the 3D position of the brush and the orientation of the face is known, to replace the user's face with a cartoon character, with augmented reality techniques.

Further characteristics and advantages of the invention will become more apparent from the following description of a preferred embodiment, illustrated by way of a non-limiting example in the figures in the attached drawings, wherein:

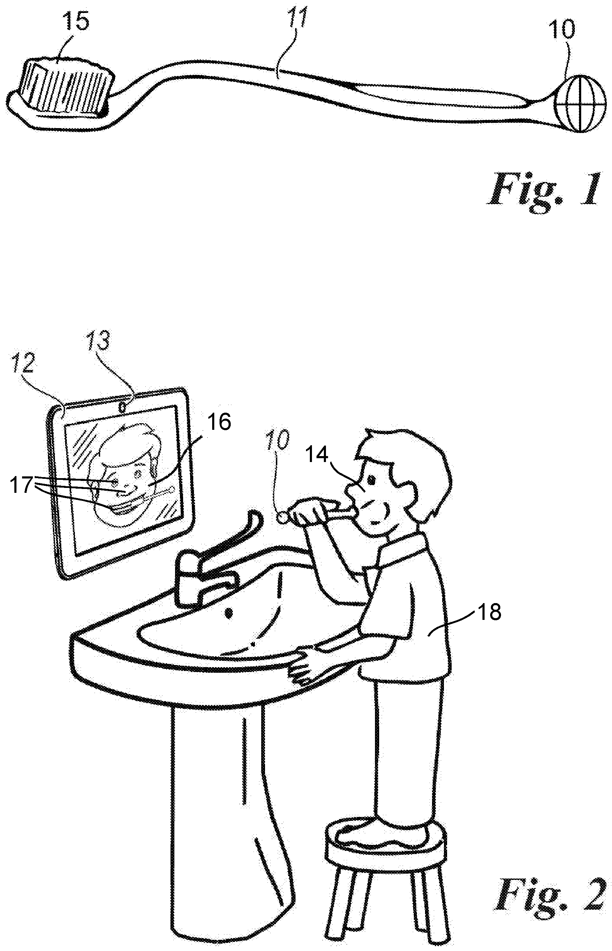

Figure 1 shows a toothbrush with a marker attached, according to the present invention;
Figure 2 shows schematically a boy brushing his teeth with a toothbrush in front of a tablet, according to the present invention;
Figure 3 shows an upper hemisphere of a marker, according to the present invention;
Figure 4 shows a lower hemisphere of a marker, according to the present invention;
Figure 5 shows a side view of a marker, according to the present invention.

A special marker 10 is fixed (by means of slotting or with glue) to the end of a toothbrush 11 (or toothbrushes with said marker already provided may be manufactured). The purpose of said marker is to allow a processing device 12, preferably a tablet, due to its dimensions and practicalness, although a computer may also be used, by means of a video camera 13 positioned on the upper part of the tablet 12, to locate the spatial position of the toothbrush 11 (orientation and 3D position), and also to analyse the direction of motion (also in space).

Also by means of a video camera 13 on the front of the tablet 12, the face 14 of the person 18 performing oral hygiene is also acquired and the image 16 is displayed on the tablet 12.

The marker 10 is placed on the toothbrush at the opposite end to its head 15.

The system is capable of locating one or more faces framed by the video camera 13 and selecting the one closest to it.

Said operation is performed by means of digital processing techniques of images forming the prior art, for example in the article by Viola P. and Jones M. J., "Robust Real-Time Face Detection" in International Journal of Computer Vision, Kluwer Academic Publishers, pages 137-f4, 2004.

In extreme summary, the image where it is presumed there is a face is filtered with a series of two-dimensional filters derived from the Haar transform on different scales, where each of said filters has been recognised in the training phase of the system as a good identifier of the face and, in particular, where different filters may be resistant to various problems in location of the face, such as occlusions or shadows, for example.

When an appropriate number of said filters gives a positive result on being applied to the image, the area filtered by them is identified as a face of which the dimensions and extension in the image are known.

Obviously, several faces may be present in an image and, in our case, only faces with sufficient dimensions and a correct tracking of the marker of the toothbrush are considered as useful. Open-source implementations of said algorithm are present in public libraries, such as OpenCV, available at http://opencv.org.

Once the face 14 is located, a series of salient parts 17 are identified, such as the eyes, nose and mouth, so as to identify the spatial position of the toothbrush 11 with respect to the mouth of the person 18 framed.

The system will be resistant to partial occlusions of the face 14 which may be due to the presence of the toothbrush 11 and the hand holding it.

In this case as well, for location of the salient parts 17 of the face 14, techniques in the prior art will be used, such as those described in the article by Cootes T.F., Edwards G.J., Taylor C.J., "Active appearance models", IEEE Transaction of Pattern Analysis and Machine Intelligence (2001).

In this case, once the position of the face (or faces) in the image is known. the Active Appearance Model algorithm will be applied to each of them, where a predefined model of the face is placed inside the area identified as containing a face. In particular, each point of the predefined model is moved along the normal direction to said model until an edge intersects on the real image. The subsequent step requires determination of the distortion parameters of the model (degrees of freedom on the mask depicting a human face) which adapt best to the new positions of the points of the model and a new configuration of the model is thus estimated. The two previous steps are then repeated in sequence until convergence is achieved. This algorithm is also available in open-source in the aforementioned OpenCV library.

The system will also be capable, if required, of recognising the toothbrush from the marker and therefore associating it with a user, in order to check that all users of the system in question clean their teeth with the times and methods set.

Once the user's mouth and orientation of their face are located, and also the position of the toothbrush by means of the applied target, temporal analysis of the spatial movement of the toothbrush is performed in a sequence of frames, in order to check that the movement of the toothbrush is the correct one.

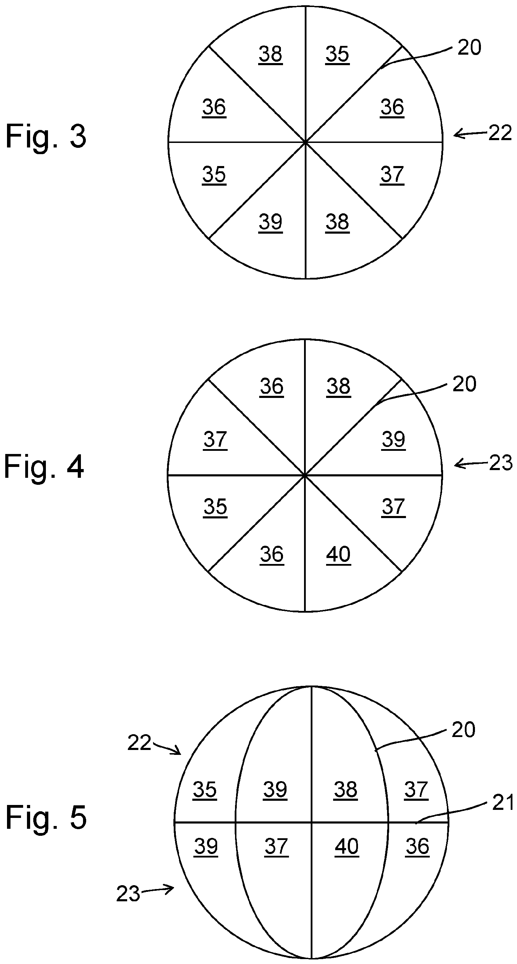

In particular, the roto-translation of the marker is known and the spatial positioning of the toothbrush head 15 is also known, as a result of the above. When the position of the mouth is known from the Active Appearance Model technique indicated above, it is easy to identify if the user is operating on the teeth on the left or the right. Furthermore, when rotation of the marker is known due to determination of the position of one or more poles (north, centre point in the circle of fig. 3, or south, centre point in the circle of fig. 4), it is possible to define the polar axis of the marker and therefore to determine whether the head 15 of the toothbrush is oriented orthogonally to the transversal plane of the mouth (the plane which separates the upper dental arc from the lower one) and therefore resting on the occlusal table of the teeth or whether the head is oriented on said plane and therefore resting on the lateral surface of the teeth.

Motion of the head is analysed by means of optical flow analysis algorithms applied to the marker at the end of the toothbrush, and in particular, the Lucas-Kanade algorithm has been chosen, indicated in the following document: B. D. Lucas and T. Kanade (1981), "An iterative image registration technique with an application to stereo vision." Proceedings of Imaging Understanding Workshop, pages 121-130.

In said differential method, assuming the intensity and the colour components of the marker in motion between the different frames is constant, and assuming that movement between two successive frames is small (a few pixels), it is assumed that, for a point considered and for the area around it, the intensities of the different colour components between two frames follow a cinematic law of uniform motion. Indicating with I one of the 3 colour components and with $\Delta x$ and $\Delta y$ the movement in pixels and with 'n' the current frame, it may be assumed that

$$I(x, y, n) = I(x + \Delta x, y + \Delta y, n + 1)$$

From which:

$$I(x + \Delta x, y + \Delta y, n + 1) = I(x, y, n) + \frac{\partial I}{\partial x}\Delta x + \frac{\partial I}{\partial y}\Delta y + \frac{\partial I}{\partial t}$$

The estimate of the speed vector of a point between two images must therefore satisfy the following equation:

$$I_x(x, y)V_x + I_y(x, y)V_y = -I_t(x, y)$$

Where $I_x$ and $I_y$ indicate the derivative in direction x and y respectively, whereas $V_x$ and $V_y$ indicate the speed components in the horizontal and vertical direction.

This operation extended to the pixels around the pixel considered allows a better rejection of noise ad a good estimate of the direction of motion of the marker to be obtained.

When the position of the mouth, the orientation of the head and the direction of its motion are known, having been estimated by analysing motion of the marker, it is possible to assess the following conditions:
Head of the toothbrush on the plane orthogonal to the transversal plane, i.e. the head resting on the occlusal table of the molars and premolars and translation on the transversal plane: said movement is correct.

Head resting on the lateral surface of the dental arcs and rotation and vertical translation: correct movement.

Head resting on the lateral surface of the dental arcs and rotation and translation on the transversal plane: incorrect movement.

Due to analysis of the spatial position of the toothbrush, it will also be possible to analyse whether both the dental arcs are affected by the cleaning activity and whether the total amount of time dedicated to cleaning corresponds with what is required for a correct oral hygiene.

The last step of the system is aimed at increasing enjoyment of the oral hygiene process for children and provides for display on the tablet 12, by means of augmented reality techniques, of an "avatar", i.e. a puppet which is superimposed on the image of the face framed, in the same spatial position and which precisely repeats the same gesture, and the image of the toothbrush itself, as its spatial position is known due to the aforementioned steps, may be depicted in augmented reality by animating it or depicting it as another object chosen by the child or associated with the avatar.

The expression of the puppet's face and the words it speaks during the tooth brushing procedure will also allow the user to understand whether the operation is being performed correctly and with the right times or whether corrections must be made.

The user has the sensation of looking into a kind of "magic mirror" where their face is mirrored in the puppet's face and the toothbrush may be transformed into another, possibly animated, object, all faithfully reflecting, in real time, the movements of the face and the toothbrush. Said aspects are aimed at attracting the user's attention and making tooth brushing an interactive and enjoyable experience.

The marker 10 is spherical and is applied to the end of the toothbrush 11, opposite the head 15. Different geometrical shapes of the marker may be used, in principle, provided that the framing of the video cameras allows the position and spatial rotation to be estimated precisely in all spatial configurations of the toothbrush during tooth brushing. It could therefore be cubic, conical or another shape; the sphere shape has been chosen since, irrespective of rotation, it always appears as a circle in the image acquired by the video camera, simplifying location, and also does not have any edges which could be unsuitable if the system is used by children.

To locate its spatial position and follow its movements, in terms of position and rotation, reference is made to a system of coordinated axes centred on the optical centre of the video camera 13.

The marker 10 has preferably been divided into 16 parts or wedges 20. In this case as well, this choice also proves to be a good compromise between the number of pixels of each part (wedges) framed on average by the video camera during the oral hygiene operation and accuracy in estimating the angles or rotation.

Other types of division of the marker aimed at determining 3D roto-translation of the toothbrush could have been adopted, such as 1D or 2D bar codes, for example, but the choice adopted also proved to be robust even in the case of acquisition with reduced dimensions of the marker framed (the marker may therefore be represented by a few hundred pixels, since it is distant from the video camera).

In the specific case, the marker has been divided into 8 meridians 20, divided in half by an equator 21 to form an upper hemisphere 22 and a lower hemisphere 23. The areas between the meridians 20 are coloured uniformly with different colours.

A two-colour marker could have been adopted (e.g. with black and white areas) but, since most video cameras used in portable devices are colour, use of a set of colours which is sufficiently discriminable both from the average background and from each other allows the density of information stored in the marker to be increased compared with two-colour markers, thus allowing uniform areas of larger dimensions which are therefore easily locatable by the video camera to be adopted.

The marker 10 is located and its roto-translation is estimated by analysing the colour components of the image acquired. In the specific case, only completely saturated colours are used for colouring of the marker, the hues of which are as distinguishable as possible with the other acquisition sensors (CMOS, CCD) with which normal tablets 12 are equipped.

Tablets perceive colour through a matrix of colour filters called the Bayer matrix, indicating with 1 the maximum intensity value detected by a cell of the sensor downstream of the Bayer colour filter and with 0 the minimum intensity.

Therefore, for each pixel, the Red, Green and Blue components of the adjacent pixels are measured or estimated for each pixel.

The colours used in creating the marker have therefore been chosen to maximise the colour contrast of the various meridians 20 and the capacity to distinguish the spherical marker from its surroundings.

Furthermore, use of colour components rather than the traditional black and white markers (bar codes or OR codes, for example) allows coding of a greater amount of information (6 possible colour variants compared with the 2 possible values of black and white).

In the specific case, the colours used are completely saturated and with the following hues: Blue 35, Red 36, Yellow 37, Green 38, Cyan 39, Magenta 40.

In a real system, there will obviously be a series of elements which reduce discriminability of the marker colours and, in order to improve recognition of each colour component, it was decided to transform the colour spaces of the R,G,B (Red Green and Blue) space into the HSV (Hue, Saturation, Value) space.

The transformation criteria to pass from the RGB colour space to the HSV one is the following. The hue is represented on a scale between 0° and 360°, conventionally starting from red at 0°, passing through green at 120° and blue at 240°, then returning to red at 360°.

Hue value H is determined with the following formula:

$$M = \max\ (R,G,B)$$

$$m = \min\ (R,G,B)$$

$$C = M - m$$

$$H' = \text{indefinite if } C = 0$$

$$H' = ((G-B)/C) \bmod 6 \text{ if } M = R$$

$$H' = ((B-R)/C)+2 \text{ if } M = G$$

$$H' = ((R-G)/C)+4 \text{ if } M = B$$

$$H = 60° \cdot H'$$

Hue value H may therefore vary between 0° and 360°.

Value V is defined with the following criterion.

$$V = M = \max (R,G,B)$$

V is therefore an approximate indicator of the intensity (radiated energy) and dark colours will have lower values.

Saturation S is calculated with the following criterion.

$$S = 0 \text{ if } C = 0$$

$$S = C/V \text{ otherwise}$$

Saturation S will therefore be zero when the three colour components of Red, Green and Blue are equal (various shades of grey) and will be higher the higher is the gap between the colour components, denoting "purer" colours, i.e. formed mainly of one or two of the components of Red, Green and Blue with respect to the third component.

The marker will be coloured with the following criteria.

1. Two consecutive areas never have the same colour.

2. The sequence of colours on each hemisphere must not have rotational symmetries with respect to the Pole, and sequences which differ solely due to cyclic rotation of the colours are therefore rejected.

3. The colours between areas bordering between the two hemispheres must be different.

4. The sequences of colours belonging to the upper hemisphere must not be confusable with those of the lower hemisphere, so that the two hemispheres are distinguishable.

5. In view of the usual distance of the marker from the video camera and considering the usually short focuses of tablets, it may be assumed that, on average, only 3 colours of the entire sequence may be distinguished and it is therefore necessary for a sequence to be recognisable and its rotation with respect to the video camera to be recognised from only 3 colours. It must therefore be possible to distinguish the hemisphere considered and its angular position from 3 colours in series.

There are 6 possible colour sequences with these restrictions. The figures show one of them, where the numerical references correspond with the relative colour.

The marker is located by means of conversion of the image from the RGB space to the HSV space As a result of this transformation, the area where the marker is present may be easily identified as an area characterised by a high saturation with a high variation in hue (due to the variation of colours defined above for definition of the marker) and, with this simple expedient, it has been possible to define a robust approach to locate the marker, irrespective of the lighting present.

The subsequent step involves recognition of the angles of rotation of the marker with respect to the system of reference of the video camera and, in particular, convection has been used to define the rotations in space through rotation of three angles in the Roll, Pitch and Yaw space.

Considering the image plane of the video camera as parallel to plane Z-Y, the rotations considered are therefore the roll (rotation on the image plane with respect to axis X), pitch (rotation with respect to vertical axis Z) and yaw (rotation with respect to horizontal axis Y).

The angles of rotation are estimated with the following procedure: once the circumference (projection of the marker sphere on the image plane) containing the marker is located with the above process, the areas (in pixel) of the different colour areas are identified, using only intensity values which are above 50% of the maximum value.

They are placed in decreasing order of area (with area being the quantity of pixels with the same hue present in the image) and the ratios between the first and the second and the second and the third areas are evaluated. The angles are then evaluated with the following criteria.

The roll angle is estimated by evaluating the position and the colour of the barycentres of the areas obtained by separating the areas of the upper hemisphere from those of the lower hemisphere: the relative angles between the different barycentres of the homogenous colour areas are estimated separately for the two hemispheres (this operation is possible since the sequences of colours of each hemisphere are known and cannot be ambiguous due to what is stated above).

For the pitch and yaw angles, by means of a series of renderings using a program of realistic generation of virtual scenes based on ray tracing, it is possible to estimate, also by varying the ratio between the focus of the camera and the ray of the marker, the ratios between the areas described above (ratio between the area of the largest area and the one just smaller than it and ratio between the latter and the third area). In particular, it is possible to place said ratios between the areas and the angles of pitch and yaw by means of a table of correspondences.

Prior knowledge of the sequence of colours and relative positioning between the marker and the toothbrush allows immediate and approximate estimation of the pitch, using the visible colours, with an error equal to 360°/8 = 45°, where "8" indicates the number of wedges used.

For a better estimate of the angles, a matrix is therefore generated, formed of 36 lines and 18 columns (corresponding with a resolution of 10° on both the pitch and the yaw), where, for each cell, 3 ratios are memorised between the prevalent colour areas, i.e. between the portions of the image of the marker which involve the highest number of pixels and the corresponding colours.

In the recognition phase, the element of the matrix which presents the ratios and colours most similar to those acquired (in terms of the Euclidean distance) is then sought and the pitch and yaw angles are thus estimated.

When the focus of the video camera in pixels is known, its distance from the video camera is also estimated from the diameter of the marker by means of a simple similarity between triangles.

Determination of the salient points of the face of the person being framed and their movements is by means of a cascade of deformable models.

There are various methods for determining the salient points of the face, as described, for example, in the article by Xiang Yu; Junzhou Huang; Shaoting Zhang; Wang Yan; Metaxas, D.N., "Pose-Free Facial Landmark Fitting via Optimized Part Mixtures and Cascaded Deformable Shape Mode, "Computer Vision (ICCV), 2013 IEEE International Conference, pp.1944,1951, 1-8 December.

In an embodiment of the present invention, a predefined number of points have been defined which identify the eyes, nose and mouth of the face in question. In particular, 6 points have been used for the mouth, 4 for each eye, 12 for the jaw line from ear to ear, 10 for the nose and 6 for each eyebrow.

In the toothbrush embodiment phase, the rotation and the distance of the marker with respect to the head must be known, in particular, since the intention is to determine the position and the movement impressed on the toothbrush head with respect to the dental arcs of the user, even though different choices may be adopted, and the north pole of the toothbrush has been aligned, the centre of the circumference in fig. 3, with the direction of the bristles of the toothbrush head.

When the position and spatial rotation of the marker placed at the end of the toothbrush, which is visible to the video camera during most of the teeth cleaning action, are known, it is possible to identify the position and spatial rotation of the head which, in contrast, is hidden in the mouth or covered by the toothpaste during most of the teeth cleaning action. When the position of the user's mouth is also known, it is possible to identify on which dental arc and on which side of the arc the toothbrush head is positioned.

The motion of the toothbrush is then analysed by an optical flow algorithm, i.e. an algorithm which is capable of determining, for a set of significant points, their motion between the different frames, thus defining a vectorial field where each vector originates in the position of the point in the first image and ends in the corresponding position in the second image.

For this purpose, there is also an approach not in the prior art, such as the one described in the article by B. Horn and B Schunck, "Determining Optical Flow" Artificial Intelligence, 16 185-203, 1981, which gives good results in view of the high separability of saturated colours of the marker from the image as a whole.

It is therefore possible to analyse the motion of the toothbrush imparted by the user for each surface of each dental arc. From joint analyses of rotation of the toothbrush and spatial orientation of the mouth, it is possible to identify on which surface of the dental arc the toothbrush bristles are resting and it is therefore possible to check whether its movement is correct.

Correct brushing is intended as a vertical movement on the vertical surfaces of the dental arc and a horizontal movement on the contact surfaces of the molars and premolars. Different or other additional procedures to the above may be provided.

Functioning of the invention is clear to a person skilled in the art from what has been described and, in particular, is as follows.

The user places the tablet 12 in front of themselves and starts the program for checking correct oral hygiene.

The video camera 13 films the image in front of it and identifies the salient points 17 on the person's face 18, particularly the mouth, but also the other points which are important for determining movements of the face. If adapted for this, the tablet 12 automatically identifies the user's face, or alternatively there could be an authentication procedure by entering the user's name.

The user is holding the toothbrush 11 and its position is therefore identified due to presence of the marker 10.

A timer starts to determine the total brushing time.

The movements of the brush and the person's face are followed to check that the relative movement between the face and the toothbrush corresponds with one of more envisaged brushing rules, such as a vertical movement for the vertical surfaces of the dental arcs and a horizontal movement for the contact surfaces of the molars and the premolars.

If movements which do not correspond with the envisaged rules are noted, acoustic/visual warnings could be provided which warn the user, or a voice could be provided which indicates to the user that brushing is incorrect and how to perform it correctly.

These events may be stored in combination with the user's name for further analysis.

On finishing brushing, the user blocks the program with a special key (virtual) on the tablet 12 and, at this point, the brushing time is calculated. This time is stored in combination with the name of the person who performed the operation.

The cleaning times and any errors are thus stored for each person.

**Claims**

1. A system for checking a correct oral hygiene procedure, **characterised by** comprising: a video camera (13) to acquire the images (16) of the face (14) of a person (18) brushing their teeth with a toothbrush (11) comprising a marker (10); processing means (12) for analysing said images (16) and locating the salient points (17) of said face (14) of a person (18); processing means (12) for analysing said images (16) and locating said toothbrush (11); processing means (12) for following the movements of said salient points (17); processing means (12) for following the movements of said toothbrush (11); processing means (12) for calculating the movement between said face (14) and said toothbrush (11); processing means (12) for checking that said movement between said face (14) and said toothbrush (11) corresponds with a relative pre-set movement; where said marker (10) has been divided into a plurality of areas (35-40); and where each of said plurality of areas (35-40) is coloured, with a colour chosen from a predefined number of colours, to maximise the colour contrast between said areas (35-40) close to each other; wherein two consecutive areas of said plurality of areas (35-40) never having the same colour; and said marker (10) being spherical and divided into a plurality of areas (35-40) defined by meridians (20) separated from each other by an equator (21); said equator (21) dividing into two hemispheres (22, 23) said marker (10); and said system identifying the hemisphere (22, 23) considered and its angular position based on recognition of three adjacent colours; said marker (10) being placed at the end of said toothbrush (11) opposite to the cleaning head (15).

2. The system according to one of the previous claims, **characterised by** comprising means of communication (12) with said person (18) brushing their teeth to warn said person (18) of an incorrect oral hygiene procedure which does not correspond with said pre-set movement.

3. The system according to one of the previous claims, **characterised by** comprising storage means (12) of a reference indicating said person (18) brushing their teeth and the time taken to do so.

4. The system according to one of the previous claims, **characterised by** comprising means for displaying (12) the face (14) of said person (18) brushing their teeth on the screen.

5. The system according to the previous claim, **characterised by** said face (14) of said person (18) brushing their teeth being replaced with another virtual face.

6. A method for checking a correct oral hygiene procedure comprising the steps of: filming with a video camera (13) the face (14) of a person (18) brushing their teeth with a toothbrush (11) comprising a marker (10); locating the salient points (17) of said face (14) of a person (18); locating said toothbrush (11); following the movements of said salient points (17); following the movements of said toothbrush (11); calculating the relative movement between said face (14) and said toothbrush (11); checking that said relative movement between said face (14) and said toothbrush (11) corresponds with a relative pre-set movement; where said toothbrush (11) comprises a spherical marker (10); dividing said marker (10) into a plurality of areas (35-40), defined by meridians (20) separated from each other by an equator (21); colouring each area (35-40) of said marker (10) to maximise the colour contrast of the different areas; wherein two consecutive areas of said plurality of areas (35-40) never having the same colour; dividing said marker (10) into two hemispheres (22, 23); and identifying the hemisphere (22, 23) and its angular position based on recognition of three adjacent colours; Placing said marker (10) at the end of said toothbrush (11) opposite the cleaning head (15).

7. A computer program adapted to carry out the method of claim 6.

**Patentansprüche**

1. System zum Prüfen einer korrekten Mundhygieneprozedur, **dadurch gekennzeichnet, dass** es Folgendes umfasst:

eine Videokamera (13) zum Erfassen der Bilder (16) des Gesichts (14) einer Person (18), die sich die Zähne mit einer Zahnbürste (11), die eine Markierung (10) umfasst, putzt; Verarbeitungsmittel (12) zum Analysieren der Bilder (16) und Lokalisieren der markanten Punkte (17) des Gesichts (14) einer Person (18); Verarbeitungsmittel (12) zum Analysieren der Bilder (16) und Lokalisieren der Zahnbürste (11), Verarbeitungsmittel (12) zum Verfolgen der Bewegungen der markanten Punkte (17); Verarbeitungsmittel (12) zum Verfolgen der Bewegungen der Zahnbürste (11); Verarbeitungsmittel (12) zum Berechnen der Bewegung zwischen dem Gesicht (14) und der Zahnbürste (11); Verarbeitungsmittel (12) zum Prüfen, dass die Bewegung zwischen dem Gesicht (14) und der Zahnbürste (11) einer relativen voreingestellten Bewegung entspricht; wo die Markierung (10) in eine Vielzahl von Bereichen (35-40) geteilt wurde und wo jeder der Vielzahl von Bereichen (35-40) farbig ist, wobei eine Farbe aus einer vordefinierten Anzahl von Farben ausgewählt wird, um den Farbkontrast zwischen den Bereichen (35-40), die nahe beieinander liegen, zu maximieren; wobei zwei aufeinanderfolgende Bereiche der Vielzahl von Bereichen (35-40) nie dieselbe Farbe haben und die Markierung (10) kugelförmig und in eine Vielzahl von Bereichen (35-40) geteilt ist, die durch Meridiane (20) definiert sind, die durch einen Äquator (21) voneinander getrennt sind; wobei der Äquator (21) die Markierung (10) in zwei Halbkugeln (22, 23) teilt und das System die in Betracht gezogene Halbkugel (22, 23) und deren Winkelposition auf Basis der Erkennung von drei benachbarten Farben identifiziert; wobei die Markierung (10) sich am Ende der Zahnbürste (11) gegenüber dem Reinigungskopf (15) befindet.

2. System nach einem der vorhergehenden Ansprüche, **gekennzeichnet durch** Umfassen eines Mittels für eine Kommunikation (12) mit der Person (18), die sich die Zähne putzt, um die Person (18) hinsichtlich einer inkorrekten Mundhygieneprozedur, die nicht der voreingestellten Bewegung entspricht, zu warnen.

3. System nach einem der vorhergehenden Ansprüche, **gekennzeichnet durch** Umfassen eines Speichermittels (12) einer Referenz, die die Person (18), die sich die Zähne putzt, und die hierfür verwendete Zeit anzeigt.

4. System nach einem der vorhergehenden Ansprüche, **gekennzeichnet durch** Umfassen eines Mittels zum Anzeigen (12) des Gesichts (14) Person (18), die sich die Zähne putzt, auf dem Bildschirm.

5. System nach dem vorhergehenden Anspruch, **dadurch gekennzeichnet, dass** das Gesicht (14) der Person (18), die sich die Zähne putzt, durch ein anderes virtuelles Gesicht ersetzt wird.

6. Verfahren zum Prüfen einer korrekten Mundhygieneprozedur, das die folgenden Schritte umfasst: Filmen des Gesichts (14) einer Person (18), die sich die Zähne mit einer Zahnbürste (11), die eine Markierung (10) umfasst, putzt, mit einer Videokamera (13); Lokalisieren der markanten Punkte (17) des Gesichts (14) einer Person (18); Lokalisieren der Zahnbürste (11); Verfolgen der Bewegungen der markanten Punkte (17); Verfolgen der Bewegungen der Zahnbürste (11); Berechnen der relativen Bewegung zwischen dem Gesicht (14) und der Zahnbürste (11); Prüfen, dass die relative Bewegung zwischen dem Gesicht (14) und der Zahnbürste (11) einer relativen voreingestellten Bewegung entspricht; wo die Zahnbürste (11) eine kugelförmige Markierung (10) umfasst; Teilen der Markierung (10) in eine Vielzahl von Bereichen (35-40), die durch Meridiane (20) definiert sind, die durch einen Äquator (21) voneinander getrennt sind; Färben jedes Bereichs (35-40) der Markierung (10), um den Farbkontrast der verschiedenen Bereiche zu maximieren; wobei zwei aufeinanderfolgende Bereiche der Vielzahl von Bereichen (35-40) nie dieselbe Farbe haben; Teilen der Markierung (10) in zwei Halbkugeln (22, 23) und Identifizieren der Halbkugel (22, 23) und deren Winkelposition auf Basis der Erkennung von drei benachbarten Farben; Platzieren der Markierung (10) am Ende der Zahnbürste (11) gegenüber dem Reinigungskopf (15).

7. Computerprogramm, das angepasst ist, das Verfahren nach Anspruch 6 durchzuführen.

**Revendications**

1. Système pour vérifier une procédure d'hygiène buccale correcte, **caractérisé en ce qu'**il comprend : une caméra vidéo (13) pour acquérir les images (16) du visage (14) d'une personne (18) se brossant les dents avec une brosse à dents (11) comprenant un marqueur (10) ; des moyens de traitement (12) pour analyser lesdites images (16) et localiser les points saillants (17) dudit visage (14) d'une personne (18) ; des moyens de traitement (12) pour analyser lesdites images (16) et localiser ladite brosse à dents (11) ; des moyens de traitement (12) pour suivre les mouvements desdits points saillants (17) ; des moyens de traitement (12) pour suivre les mouvements de ladite brosse à dents (11) ; des moyens de traitement (12) pour calculer le mouvement entre ledit visage (14) et ladite brosse à dents (11) ; des moyens de traitement (12) pour vérifier que ledit mouvement entre ledit visage (14) et ladite brosse à dents (11) correspond à un mouvement relatif prédéfini ; où ledit marqueur (10) a été divisé en une pluralité de zones (35-40) ; et où chacune de ladite pluralité de zones (35-40) est colorée avec une couleur choisie parmi un nombre prédéfini de couleurs, afin de maximiser le contraste de couleurs entre lesdites zones (35-40) proches les unes aux autres ; où deux zones consécutives de ladite pluralité de zones (35-40) n'ont jamais la même couleur ; et ledit marqueur (10) étant sphérique et divisé en une pluralité de zones (35-40) définies par des méridiens (20) séparés les uns des autres par un équateur (21) ; ledit équateur (21) divisant en deux hémisphères (22, 23) ledit marqueur (10) ; et ledit système identifiant l'hémisphère (22, 23) considéré et sa position angulaire sur la base de la reconnaissance de trois couleurs adjacentes ; ledit marqueur (10) étant placé à l'extrémité de ladite brosse à dents (11) opposée à la tête de nettoyage (15).

2. Système selon l'une des revendications précédentes, **caractérisé en ce qu'**il comprend des moyens de communication (12) avec ladite personne (18) se brossant les dents pour avertir ladite personne (18) d'une procédure d'hygiène buccale incorrecte ne correspondant pas audit mouvement prédéfini.

3. Système selon l'une des revendications précédentes, **caractérisé en ce qu'**il comprend des moyens de stockage (12) d'une référence indiquant que ladite personne (18) est en train de se brosser les dents et le temps nécessaire pour le faire.

4. Système selon l'une des revendications précédentes, **caractérisé en ce qu'**il comprend des moyens pour montrer (12) le visage (14) de ladite personne (18) se brossant les dents sur l'écran.

5. Système selon la revendication précédente, **caractérisé en ce que** ledit visage (14) de ladite personne (18) se brossant les dents est remplacé par un autre visage virtuel.

6. Méthode pour vérifier une procédure d'hygiène buccale correcte comprenant les étapes consistant à : filmer avec une caméra vidéo (13) le visage (14) d'une personne (18) se brossant les dents avec une brosse à dents (11) comprenant un marqueur (10) ; localiser les points saillants (17) dudit visage (14) d'une personne (18) ; localiser ladite brosse à dents (11) ; suivre les mouvements desdits points saillants (17) ; suivre les mouvements de ladite brosse à dents (11) ; calculer le mouvement relatif entre ledit visage (14) et ladite brosse à dents (11) ; vérifier que ledit mouvement relatif entre ledit visage (14) et ladite brosse à dents (11) correspond à un mouvement relatif prédéfini ; où ladite brosse à dents (11) comprend un marqueur sphérique (10) ; diviser ledit marqueur (10) en une pluralité de zones (35-40), définies par des méridiens (20) séparés les uns des autres par un équateur (21) ; colorer chaque zone (35-40) dudit marqueur (10) afin de maximiser le contraste de couleur des différentes zones; où deux zones consécutives de ladite pluralité de zones (35 à 40) n'ont jamais la même couleur ; diviser ledit marqueur (10) en deux hémisphères (22, 23) ; et identifier l'hémisphère (22, 23) et sa position angulaire sur la base de la reconnaissance de trois couleurs adjacentes ; placer ledit marqueur (10) à l'extrémité de ladite brosse à dents (11) opposée à la tête de nettoyage (15).

7. Programme informatique adapté pour mettre en oeuvre la méthode de la revendication 6.

Fig. 1

Fig. 2

Fig. 3

38  35

36  36

35  37

39  38

20

22

Fig. 4

36  38

37  39

35  37

36  40

20

23

Fig. 5

22

35  39  38  37

39  37  40  36

20

21

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ANNA FLAGG et al.** An Intelligent Toothbrush: Machines for Smart Brushing. *Rehabilitation Engineering and Assistive Technology Society of North America (RESNA),* 05 June 2011
- **YU-CHEN CHANG et al.** Playful Toothbrush: Ubi-Comp Technology for Teaching Tooth Brushing to Kindergarten Children. *26th Annual CHI Conference on Human Factors in Computing Systems, CHI 2008 : conference proceedings,* 05 April 2008
- Robust Real-Time Face Detection. **VIOLA P. ; JONES M. J.** International Journal of Computer Vision. Kluwer Academic Publishers, 2004, 137-f4
- **COOTES T.F. ; EDWARDS G.J. ; TAYLOR C.J.** Active appearance models. *IEEE Transaction of Pattern Analysis and Machine Intelligence,* 2001
- **B. D. LUCAS ; T. KANADE.** An iterative image registration technique with an application to stereo vision. *Proceedings of Imaging Understanding Workshop,* 1981, 121-130
- **XIANG YU ; JUNZHOU HUANG ; SHAOTING ZHANG ; WANG YAN ; METAXAS, D.N.** Pose-Free Facial Landmark Fitting via Optimized Part Mixtures and Cascaded Deformable Shape Mode. *Computer Vision (ICCV), 2013 IEEE International Conference,* 1944
- **B. HORN ; B SCHUNCK.** Determining Optical Flow. *Artificial Intelligence,* 1981, vol. 16, 185-203